# EUROPEAN PATENT SPECIFICATION

(11) **EP 4 326 305 B1**
(45) Date of publication and mention of the grant of the patent: **15.07.2026**
(21) Application number: 22723414.3
(22) Date of filing: 19.04.2022
(51) Int. Cl.: A61K 38/08, A61K 38/06, A61K 8/64, A61P 17/14, A61Q 5/00

(54) **ACETYL SH-HEXAPEPTIDE-5 AMIDE ACETATE FOR USE IN THE TREATMENT OF HAIR LOSS DISORDERS AND AS A HAIR CARE AGENT**
ACETYL-SH-HEXAPEPTID-5-AMID-ACETAT ZUR VERWENDUNG IN DER BEHANDLUNG VON HAARAUSFALLERKRANKUNGEN UND ALS HAARPFLEGEMITTEL
ACÉTATE D'ACÉTYLE SH-HEXAPEPTIDE-5 AMIDE DESTINÉ À ÊTRE UTILISÉ DANS LE TRAITEMENT DE TROUBLES DE LA PERTE DE CHEVEUX ET EN TANT QU'AGENT DE SOINS CAPILLAIRES

(30) Priority: 20.04.2021 EP 21382340
(43) Date of publication of application: 28.02.2024
(73) Proprietor: Laboratorio Genove, S.A., 08907 l'Hospitalet de Llobregat (ES)
(72) Inventor: FLORIACH GUAL, Nuria, 08907 L'HOSPITALET DE LLOBREGAT (ES); ARIAS CHOUSA, Eva Maria, 08907 L'HOSPITALET DE LLOBREGAT (ES)
(74) Representative: ZBM Patents - Zea, Barlocci & Markvardsen
(86) International application number: PCT/EP2022/060259
(87) International publication number: WO 2022/223525

(56) References cited:
- KISHIMOTO J ET AL: "SELECTIVE ACTIVATION OF THE VERSICAN PROMOTER BY EPITHELIAL-MESENCHYMAL INTERACTIONS DURING HAIR FOLLICLE DEVELOPMENT", PROCEEDINGS OF THE NATIONAL ACADEMY OF SCIENCES, NATIONAL ACADEMY OF SCIENCES, US, vol. 96, 1 June 1999 (1999-06-01), pages 7336 - 7341, XP002925309, ISSN: 0027-8424, DOI: 10.1073/PNAS.96.13.7336
- IINO MASATO ET AL: "Adenosine Stimulates Fibroblast Growth Factor-7 Gene Expression Via Adenosine A2b Receptor Signaling in Dermal Papilla Cells", JOURNAL OF INVESTIGATIVE DERMATOLOGY, ELSEVIER, NL, vol. 127, no. 6, 1 June 2007 (2007-06-01), pages 1318 - 1325, XP009144510, ISSN: 0022-202X, [retrieved on 20070215]
- LIPOTRUE: "Lipotrue Catalogue 2019", 1 January 2019 (2019-01-01), pages 1 - 4, XP055843892, Retrieved from the Internet <URL:https://www.prodottigianni.com/uplFiles/files/Cosmesi/Lipotrue_Catalogue%202019.pdf> [retrieved on 20210922]
- PUIG A ET AL: "A new decorin-like tetrapeptide for optimal organization of collagen fibres", JOURNAL OF COSMETIC SCIENCE, SOCIETY OF COSMETIC CHEMISTS, NEW YORK, NY, US, vol. 30, 1 January 2008 (2008-01-01), pages 97 - 104, XP007914942, ISSN: 1525-7886

## Description

This application claims the benefit of European Patent Application EP21382340.4 filed on April 20th, 2021.

The present invention relates to the field of pharmacy and cosmetics. In particular, it refers to the use of acetyl sh-hexapeptide-5 amide acetate, alone or in combination with tripeptide-10 citrulline in the treatment of hair loss in a subject suffering from a hair loss disorder, or alternatively as hair care agent in a subject not suffering from a hair loss. The invention also refers to pharmaceutical and cosmetic compositions comprising the acetyl sh-hexapeptide-5 amide acetate and tripeptide-10 citrulline.

### Background Art

Hair is a symbol of vitality and health, as well as a fundamental characteristic of a person's individuality and identity. The treatment of the hair and the scalp and, in particular, the treatment and prevention of hair loss, seborrhea, dandruff, dermatitis as well as the improvement of thickness or volume has been a frequent problem of consultation for many years to dermatologists, for both men and women.

Hair is an appendage of the skin that includes an external part, or hair shaft, and an internal part, called the hair follicle. Within the follicle, different parts can be distinguished: the follicular matrix, where the hair will be created, and the sebaceous gland that will lubricate the stem on its way out. Each hair follicle is subjected to a number of hair growth cycles. These cycles consist of three distinct phases: a phase of growth and formation of the hair shaft (called anagen phase), followed by a regression phase based on cell apoptosis of the matrix (called catagen phase) and, finally, a period of rest of the hair follicle (called telogen phase). In summary, the hair cycle is divided into 3 phases: the anagen phase (or growth phase); the catagen phase (where growth slows and hair begins to separate from the dermal papilla); and the telogen phase (it is the shedding phase in which the hair is expelled to the outside).

Hair falls out can be considered "normal" as part of the body's natural renewal cycle. The American Academy of Dermatology has been noted that it is normal for a person to lose about 50-100 hairs each day. However, hair loss disorder involves a higher (abnormal) hair falls. Abnormal hair loss is comonly caused or induced by an alteration in hair growth cycles due to a multitude factors such as the impact of androgens and their genetic receptors, skin changes or stress, among others. These alterations in the capillary cycle mainly involve a reduction in the number of hairs in the anagen phase and an increase in the number of hairs in the telogen phase; as well as a shortening of the anagen phase. The consequence of these cycle's modifications means that the hair cannot grow balanced for the necessary time and then, the hair follicles become progressively smaller and the hair become shorter and thinner, while falling prematurely.

Growth and differentiation of postnatal hair follicles are controlled by reciprocal interactions between the dermal papilla and the surrounding epidermal hair precursors. The molecular nature of these interactions is largely unknown, but they are likely to involve several families of signalling molecules, including activators as well as inhibitors, for instance, Fibroblast Growth Factors (FGFs), Noggin (NOG) and bone morphogenetic proteins (BMPs). The length and size of hair depend on the anagen term in its hair cycle. It has been reported that some cell growth factors, including VEGF, IGF-1, FGF1 and FGF-5S, induce the proliferation of cells in the matrix, dermal papilla, and dermal papillary vascular system. On the other hand, negative factors like FGF-5, TGF-β1, or still unknown ones, terminate the anagen phase.

Specifically, FGF7 (Fibroblast Growth Factor 7), also known as Heparin-binding growth factor 7, is a member of the fibroblast growth factor family with broad mitogenic and cell survival activities, involved in a variety of biological processes, including embryonic development, cell growth, morphogenesis, tissue repair, tumour growth and invasion. This protein modified the endothelial cell migration and proliferation, as well as an angiogenic factor. In particular, FGF7 is highly expressed during the hair growth cycle; meanwhile in the cycle of normal hair follicles, the expression of FGF-7 will stop increasing to avoid the overgrowth of hair.

The publication "Adenosine stimulates fibroblast growth factor-7 gene expression via adenosine A2b receptor signaling in dermal papilla cells", Journal of Investigative Dermatology 2007, Jun ;127(6) :1318-25, discloses a study to investigate the underlying mechanisms on the adenosine receptor-mediated signal-transduction pathway which contributes to minoxidil-induced hair growth. By conducting DNA microarray analysis on Dermal Papilla Cells (DPCs), the results show that adenosine upregulates FGF-7 at mRNA and protein levels. To further understand the contribution of FGF-7 during hair growth, the study also assays the effects of human FGF-7 protein and concludes that FGF-7 may stimulate hair growth by inducing the proliferation and differentiation of human hair follicular cells.

In the same way, Insulin-like growth factor 1 (IGF1) has been identified as an important growth factor in many biological systems. Recently, it has been suggested that it plays a role in regulating cellular proliferation and migration during the development of hair follicles. IGF1 has an essential role in hair cycle control, as well as hair shaft differentiation during the development of hair follicles. It has been disclosed that IGF1 promotes hair follicle growth in vitro by regulating cellular proliferation. In particular, it has been observed that the absence of IGF-1 or insulin, in hair follicles in organ culture promotes the entrance of hair in catagen phase. And, furthermore, IGF-1 receptor knockout mice show a marked decrease in the absolute number of hair follicles, abnormal hair follicular pattern and hair differentiation. Therefore, IGF has been disclosed as a candidate for androgen-induced hair growth factor.

Finally, antigen KI-67 also known as Ki-67 or MKI67 (Marker of Proliferation Ki-67) is a protein that is encoded by the MKI67 gene in humans. The Ki-67 protein is a cellular marker for proliferation and can be used in immunohistochemistry. It is strictly associated with cell proliferation. During interphase, the Ki-67 antigen can be exclusively detected within the cell nucleus, whereas in mitosis most of the protein is relocated to the surface of the chromosomes. In fact, Ki-67 protein is present during all active phases of the cell cycle (G1, S, G2, and mitosis), but is absent in resting (quiescent) cells (G0).

Many compounds have been disclosed in the state of the art for the treatment and/or prevention of diseases or disorders of the hair, particularly for the abnormal hair loss, such as minoxidil for example. Most of these compounds try to normalized the hair cycles adjusting or increasing the anagen phase. Unfortunately, these treatments involve the apperance of many side effects associated with their topical application such as burning, irritation, itching and redness of the treated area of the scalp, as well as the growth of unwanted hair anywhere on the body.

On the other hand, other compounds have been also disclosed for improving the general conditions of the skin/scalp and restoring the barrier function of the skin/scalp and reducing inflammatory reactions. However, these compounds are used in treatments that are long and tedious involving months. Furthermore, the beneficial effect of these treatment is observed in a long-term period, that is also about months. This is disadvantegeous because it causes a significant increase of the dropouts due to not observing a hair benefit in a short period of time, increasing therefore, the abandonment of the treatment.

In order to increase the compliance and the reduction of the time period for observing a beneficial effect, combined treatments have been disclosed. Typically, the use of different compositions with different regimen and routes of administration has been disclosed. In particular, a combined topical treatment with an oral treatment; or even with rinsing cleaning compositions are commonly used for months for having a dual short- and long-term hair effect. However, even the benefits of the combined treatment, they do not compensate the complexity of their use mode, involving a low percentage of adherence to the treatment.

Thus, it can be concluded that the low effectiveness and the side effects associated with the treatment, in combination with the complexity of their administration/application are the main cause of the lack of compliance by the patient, causing a high percentage of treatment abandonment.

Therefore, from what is known in the state of the art, there is still the need to develop a more efficient and simple treatment for hair disorders as well as a cosmetic use for an effective hair care with fewer or no side effects.

### Summary of Invention

Inventors have found that acetyl sh-hexapeptide-5 amide acetate allows increasing the gene expression of Fibroblast Growth Factor-7 (FGF7), Insulin-like Growth Factor 1 (IGF1), and Ki67 (MKI67) and therefore, acetyl sh-hexapeptide-5 amide acetate is useful as pharmaceutical active ingredient for the treatment of hair loss in a subject suffering from a hair loss disorder; and also as hair care agent in a subject not suffering from a hair loss disorder, because it is capable of ameliorate symptoms such as hair brittleness, hair thinning, hair breakage, lack of hair elasticity, lack of hair brightness, lack of hair volume, hair roughness, hair flaking, hair dehydration, reduction of hair density and hair dryness.

Furthermore, the inventors have also found that the combined therapy of acetyl sh-hexapeptide-5 amide acetate with one or more additional active ingredients, such as tripeptide-10 citrulline, are also useful as pharmaceutical active ingredient and also as hair care agent as disclosed above. In particular, the combined therapy implies: (i) the use of acetyl sh-hexapeptide-5 amide acetate with indications of being used in combination with other active ingredients, such as tripeptide-10 citrulline; or alternatively, (ii) the use of one single unit dosage form (unique composition) comprising acetyl sh-hexapeptide-5 amide acetate with one or more additional active ingridients, such as tripeptide-10 citrulline; or alternatively, (iii) the use of two different dosage forms containing one of them the acetyl sh-hexapeptide-5 amide acetate; and the other(s) one or more additional active ingridients, such as tripeptide-10 citrulline, wherein the different dosage forms can be administered simultaneously, sequentially or separately. Regardless the administration regimen, the effectiveness of the treatmnent is maintained.

Particularly, the acetyl sh-hexapeptide-5 amide acetate and the additional active ingredients, such as tripeptide-10 citrulline, can be in form of liposomes (such as lecithin liposomes) or in free form. In both forms, the effectiveness of the compounds has been also demonstrated, being significantly increased with the topical use of the liposomes of acetyl sh-hexapeptide-5 amide acetate and liposomes of tripeptide-10 citrulline. Furthermore, it is also part of the invention a composition comprising a pharmaceutically or cosmetically effective amount of acetyl sh-hexapeptide-5 amide acetate and a pharmaceutically or cosmetically effective amount of with one or more additional active ingridients, such as tripeptide-10 citrulline.

Thus, the first aspect fo the invention refers to cetyl sh-hexapeptide-5 amide acetate, or in form of liposomes; or a pharmaceutical composition comprising the therapeutically effective amount of acetyl sh-hexapeptide-5 amide acetate, or in form of liposomes together with one or more pharmaceutically acceptable excipients or carriers; for use in the treatment of hair loss in a subject suffering from a hair loss disorder.

The second aspect of the invention refers to acetyl sh-hexapeptide-5 amide acetate, or in form of liposomes, for use in the treatment of hair loss, wherein the acetyl sh-hexapeptide-5 amide acetate, or in form of liposomes, is for use in combination therapy with tripeptide-10 citrulline, or in form of liposomes.

The third aspect of the invention refers to acetyl sh-hexapeptide-5 amide acetate, or in form of liposomes; and tripeptide-10 citrulline, or in form of liposomes, for use in the treatment of hair loss.

The fourth aspect of the invention refers to cosmetic use of acetyl sh-hexapeptide-5 amide acetate, or in form of liposomes, or of a cosmetic composition comprising a cosmetically effective amount of acetyl sh-hexapeptide-5 amide acetate together with one or more cosmetically acceptable excipients or carriers; as hair care agent in a subject not suffering from a hair loss disorder, wherein the hair care comprises ameliorating at least one of the following symptoms: hair brittleness, hair thinning, hair breakage, lack of hair elasticity, lack of hair brightness, lack of hair volume, hair roughness, hair flaking, hair dehydration, reduction of hair density and hair dryness.

The fifth aspect of the invention refers to cosmetic use of acetyl sh-hexapeptide-5 amide acetate, or in form of liposomes, as hair care agent in a subject not suffering from a hair loss disorder; in combination therapy with tripeptide-10 citrulline, or in form of liposomes; wherein the hair care comprises ameliorating at least one of the following symptoms: hair brittleness, hair thinning, hair breakage, lack of hair elasticity, lack of hair brightness, lack of hair volume, hair roughness, hair flaking, hair dehydration, reduction of hair density and hair dryness.

The sixth aspect of the invention refers to the cosmetic use of acetyl sh-hexapeptide-5 amide acetate, or in form of liposomes; and tripeptide-10 citrulline, or in form of liposomes, as hair care agent in a subject not suffering from a hair loss disorder; wherein the hair care comprises ameliorating at least one of the following symptoms: hair brittleness, hair thinning, hair breakage, lack of hair elasticity, lack of hair brightness, lack of hair volume, hair roughness, hair flaking, hair dehydration, reduction of hair density and hair dryness.

And, the seventh aspect of the invention refers to a pharmaceutical or cosmetic composition comprising: a therapeutically or cosmetically effective amount of acetyl sh-hexapeptide-5 amide acetate, or in form of liposomes; and a therapeutically or cosmetically effective amount of tripeptide-10 citrulline, or in form of liposomes;
together with one or more pharmaceutically or cosmetically acceptable excipients or carriers.

### Detailed description of the invention

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition.

For the purposes of the present invention, any ranges given include both the lower and the upper endpoints of the range. Ranges given, such as weight, temperatures, times, weights, and the like, should be considered approximate, unless specifically stated.

The terms "percentage (%) by weight", "weight/weight %" and "w/w%" have the same meaning and are used interchangeable. They refer to the percentage of each ingredient of the composition in relation to the total weight of the composition.

As mentioned above, the first aspect of the invention refers to the use of acetyl sh-hexapeptide-5 amide acetate, or in form of liposomes, or a pharmaceutical composition comprising the terapeutically effective amount of acetyl sh-hexapeptide-5 amide acetate together with one or more pharmaceutically acceptable excipients or carriers, for use in the treatment of hair loss in a subject suffering from a hair loss disorder.

The acetyl sh-hexapeptide-5 amide acetate is the INCI name of the acetate salt of the product obtained by the acetylation of sh-Hexapeptide-5 SP in which the C-terminus of the peptide is an amide. The acetyl sh-hexapeptide-5 amide acetate is commercially available with the name of versillin further containing water and phenoxyethanol (lipotrue^{®}).

The term an "effective amount" of an active ingredient refers to the amount of the active ingredient that provides the therapeutic and/or cosmetic effect after its application/administration. The term "therapeutically effective amount" as used herein, refers to the amount of an active ingredient for instance acetyl sh-hexapeptide-5 amide acetate, or tripepetide-10 citrulline that, when administered/applied, is sufficient to prevent development of, or alleviate to some extent, one or more of the symptoms of the disease which is addressed. The particular dose of the active ingredient administered according to this invention will be determined by the particular circumstances surrounding the case, including the compound administered, the route of administration, the particular condition being treated, and the similar considerations. The therapeutical use involves the administration/application of a therapeutically effective amount of acetyl sh-hexapeptide-5 amide acetate for the treatment of hair loss in a subject suffering from a hair loss disorder. For the purpose of the present invention, the term "treat" encompasses decrease, suppress, attenuate, diminish, arrest, or stabilize the development or progression of a disorder, lessen the severity of the disorder, or reduce the symptoms associated with the disorder. The term "hair loss disorder" refers to any condition that involves an excessive or abnormal loss of hair on one or more areas of the body, particularly in the head (scalp). In an embodiment, the hair loss disorder is selected from the group consisting of androgenetic alopecia, alopecia areata, telogen effluvium, alopecia totalis, alopecia universalis, alopecia senile and alopecia caused by seborrheic dermatitis. In an embodiment, the treatment of a hair loss disorder is carried out by re-growing of the hair, by prevention of further hair loss, by diminishing the rate of hair loss, by reducing the telogen phase and increasing the anagen phase and/or by enzimatic inhibition of 5-alpha reductase enzime. As it is demonstrated in the experimental section of the present application, the acetyl sh-hexapeptide-5 amide acetate alone or in combination therapy with tripeptide-10 citrulline are capable of increase the gene expression of Fibroblast Growth Factor-7 (FGF7), Insulin-like Growth Factor-1 (IGF1) and Marker of Proliferation KI-67 (MKI67) which are involved in the cell proliferation of the hair follicles and therefore, involved in the treat of hair loss.

As it is mentioned above, the acetyl sh-hexapeptide-5 amide acetate is useful for the treatment of hair loss disordes either in free form or in form of liposome. In an embodiment, the acetyl sh-hexapeptide-5 amide acetate is in free form. In an embodiment, the acetyl sh-hexapeptide-5 amide acetate is in form of liposome. For the purpose of the invention, the term "liposome" refers to a spherical vesicle having at least one lipid bilayer. Liposomes are most often composed of phospholipids, especially phosphatidylcholine, but may also include other lipids, such as egg phosphatidylethanolamine, so long as they are compatible with lipid bilayer structure. The major types of liposomes are the multilamellar vesicle (MLV, with several lamellar phase lipid bilayers), the small unilamellar liposome vesicle (SUV, with one lipid bilayer), the large unilamellar vesicle (LUV), and the cochleate vesicle. A less desirable form are multivesicular liposomes in which one vesicle contains one or more smaller vesicles. The term "liposomes" does not encompasses micelles and reverse micelles composed of monolayers instead of bilayers. In an embodiment, the acetyl sh-hexapeptide-5 amide acetate is in form of lecithin liposomes. The term lecithin refers to a generic term to designate any group of yellow-brownish fatty substances occurring in animal and plant tissues which are amphiphilic composed of a mixture of glycerophospholipids including phosphatidylcholine, phosphatidylethanolamine, phosphatidylinositol, phosphatidylserine, and phosphatidic acid. The process for the preparation of liposomes of acetyl sh-hexapeptide-5 amide acetate can readily be determined by those skilled in the art from those disclosed in the state of the art according to the type of formulation being prepared. Typically, the process for the preparation of liposomes implies mixing the phospholipids (such as lecithin) with the acetyl sh-hexapeptide-5 amide acetate and the mixture obtained is diluted in an aqueous medium.

Further, the first aspect of the invention also refers to a pharmaceutical composition comprising the therapeutically effective amount of acetyl sh-hexapeptide-5 amide acetate, or in form of liposomes, together with one or more pharmaceutically acceptable excipients or carriers; for use in the treatment of hair loss in a subject suffering from a hair loss disorder. All the embodiments, disclosed above for the compositions of the present invention also apply here for the composition for use of the first aspect of the invention.

The second aspect of the invention refers to acetyl sh-hexapeptide-5 amide acetate, or in form of liposomes, for use in the treatment of hair loss in a subject suffering from a hair loss disorder, wherein the acetyl sh-hexapeptide-5 amide acetate, or in form of liposomes, is for use in combination therapy with tripeptide-10 citrulline, or in form of liposomes. It means that the treatment of hair loss disorder involve the use of acetyl sh-hexapeptide-5 amide acetate and indications for the use of acetyl sh-hexapeptide-5 amide acetate, or in form of liposomes, in combined therapy with tripeptide-10 citrulline, or in form of liposomes.

The third aspect of the invention refers to acetyl sh-hexapeptide-5 amide acetate, or in form of liposomes; and tripeptide-10 citrulline, or in form of liposomes, for use in the treatment of hair loss in a subject suffering from a hair loss disorder.

In an embodiment, the use of acetyl sh-hexapeptide-5 amide acetate, or in form of liposomes; and tripeptide-10 citrulline, or in form of liposomes, in the treatment of hair loss in a subject suffering from a hair loss disorder, is one wherein the treatment comprises administering to a subject:
either (a) a pharmaceutical composition comprising:
a therapeutically effective amount of acetyl sh-hexapeptide-5 amide acetate, or in form of liposomes; and
a therapeutically effective amount of tripeptide-10 citrulline, or in form of liposomes;
together with one or more pharmaceutically acceptable excipients or carriers;
or alternatively, (b) simultaneously, sequentially or separately:
   (b1) a pharmaceutical composition comprising a therapeutically effective amount of acetyl sh-hexapeptide-5 amide acetate, or in form of liposomes; together with one or more pharmaceutically acceptable excipients or carriers; and
   (b2) a pharmaceutical composition comprising a therapeutically effective amount of tripeptide-10 citrulline, or in form of liposomes; together with one or more pharmaceutically acceptable excipients or carriers.

In an embodiment, the acetyl sh-hexapeptide-5 amide acetate, or in form of liposomes; and tripeptide-10 citrulline, or in form of liposomes, for use in the treatment of hair loss in a subject suffering from a hair loss disorder is one wherein the treatment comprises administering to a subject: (a) a pharmaceutical composition comprising: a therapeutically effective amount of acetyl sh-hexapeptide-5 amide acetate, or in form of liposomes; and a therapeutically effective amount of tripeptide-10 citrulline or in form of liposomes; together with one or more pharmaceutically acceptable excipients or carriers. It means that the acetyl sh-hexapeptide-5 amide acetate and the tripeptide-10 citrulline are in a single unit dosage form. The term "single unit" encompasses one entity such as a single tablet, a single granule, a single pellet, and a single ampoule. The term "single unit dosage form" defines a dosage form which consists only of one unit which contains the effective amount of the active ingredients, particularly acetyl sh-hexapeptide-5 amide acetate, or in form of liposomes, and/or the tripeptide-10 citrulline, or in form of liposomes.

In an embodiment, the acetyl sh-hexapeptide-5 amide acetate, or in form of liposomes; and tripeptide-10 citrulline, or in form of liposomes, for use in the treatment of hair loss in a subject suffering from a hair loss disorder is one wherein the treatment comprises administering to a subject: (b) simultaneously, sequentially or separately: (b1) a pharmaceutical composition comprising a therapeutically effective amount of acetyl sh-hexapeptide-5 amide acetate, or in form of liposomes; together with one or more pharmaceutically acceptable excipients or carriers; and (b2) a pharmaceutical composition comprising a therapeutically effective amount of tripeptide-10 citrulline or in form of liposomes; together with one or more pharmaceutically acceptable excipients or carriers. It means that the acetyl sh-hexapeptide-5 amide acetate, or in form of liposomes; and tripeptide-10 citrulline, or in form of liposomes, are in different single unit dosage forms which can be administered simultaneously, sequentially or separately.

For the purpose of the invention, the term tripeptide-10 citrulline is the INCI name of the following amino acid sequence: L-Ornithinamide, L-lysyl-L-alpha-aspartyl-L-isoleucyl-N5-(aminocarbonyl)-, which has the CAS number 960531-53-7. The tripeptide-10 citrulline is commercially available in form of lecithin liposomes with the name of Decorinyl^{®} which further contains water, lecithin, carbomer, triethanolamine, caprylyl glycol and phenoxyethanol.

As it is mentioned above, the tripeptide-10 citrulline is useful for the treatment of hair loss disordes either in free form or in form of liposome. In an embodiment, the tripeptide-10 citrulline is in free form. In an embodiment, the tripeptide-10 citrulline is in form of liposome. In an embodiment, the acetyl sh-hexapeptide-5 amide acetate is in form of lecithin liposomes. The process for the preparation of liposomes of tripeptide-10 citrulline can readily be determined by those skilled in the art from those disclosed in the state of the art according to the type of formulation being prepared. Typically, the process for the preparation of liposomes implies mixing the phospholipids (such as lecithin) with the acetyl sh-hexapeptide-5 amide acetate and the mixture obtained is diluted in an aqueous medium.

In an embodiment, the acetyl sh-hexapeptide-5 amide acetate is in free form and the tripeptide-10 citrulline is in free form. In an embodiment, the acetyl sh-hexapeptide-5 amide acetate is in form of liposomes, lecithin liposomes, and the tripeptide-10 citrulline is in form of liposomes, lecithin liposomes. In an embodiment, the acetyl sh-hexapeptide-5 amide acetate is in free form and the tripeptide-10 citrulline is in form of liposomes, lecithin liposomes. In an embodiment, the acetyl sh-hexapeptide-5 amide acetate is in form of liposomes, lecithin liposomes, and the tripeptide-10 citrulline is in free form. In an embodiment, the acetyl sh-hexapeptide-5 amide acetate is in form of liposomes and the tripeptide-10 citrulline is in form of liposomes. In an embodiment, the acetyl sh-hexapeptide-5 amide acetate is in form of lecithin liposomes and the tripeptide-10 citrulline is in form of lecithin liposomes.

The above-mentioned "compound for use" aspects could be also formulated as the use of acetyl sh-hexapeptide-5 amide acetate, or in form of liposomes, or a pharmaceutical composition comprising the therapeutically effective amount of acetyl sh-hexapeptide-5 amide acetate, or in form of liposomes, together with one or more pharmaceutically acceptable excipients or carriers; in the preparation of a medicament for the treatment of hair loss in a subject suffering from a hair loss disorder. Use of acetyl sh-hexapeptide-5 amide acetate, or in form of liposomes, in the preparation of a medicament for the treatment of hair loss in a subject suffering from a hair loss disorder, wherein said medicament is for use in combination therapy with tripeptide-10 citrulline, or in form of liposomes. Use of acetyl sh-hexapeptide-5 amide acetate, or in form of liposomes, and tripeptide-10 citrulline, or in form of liposomes, in the preparation of a medicament for the treatment of hair loss in a subject suffering from a hair loss disorder. Use of acetyl sh-hexapeptide-5 amide acetate, or in form of liposomes, in the preparation of a medicament for the treatment of hair loss in a subject suffering from a hair loss disorder, wherein said treatment comprises administering to a subject simultaneously, sequentially or separately tripeptide-10 citrulline, or in form of liposomes, and acetyl sh-hexapeptide-5 amide acetate, or in form of liposomes.

The above-mentioned "compound for use "aspects could be also formulated as a method for the treatment of a subject suffering or susceptible to suffer from a hair loss disorder, wherein the method comprises administering to said subject an effective amount of acetyl sh-hexapeptide-5 amide acetate, or in form of liposomes, or a pharmaceutical composition comprising the therapeutically effective amount of acetyl sh-hexapeptide-5 amide acetate, or in form of liposomes, together with one or more pharmaceutically acceptable excipients or carriers. A method for the treatment of a subject suffering or susceptible to suffer from a hair loss disorder, wherein the method comprises administering to said subject an effective amount of acetyl sh-hexapeptide-5 amide acetate, or in form of liposomes, or a pharmaceutical composition comprising the therapeutically effective amount of acetyl sh-hexapeptide-5 amide acetate, or in form of liposomes, together with one or more pharmaceutically acceptable excipients or carriers in combination therapy with tripeptide-10 citrulline, or in form of liposomes. A method for the treatment of a subject suffering or susceptible to suffer from a hair loss disorder, wherein the method comprises administering to said subject an effective amount of acetyl sh-hexapeptide-5 amide acetate, or in form of liposomes, and tripeptide-10 citrulline, or in form of liposomes; or a pharmaceutical composition comprising the therapeutically effective amount of acetyl sh-hexapeptide-5 amide acetate, or in form of liposomes, and the therapeutically effective amount of tripeptide-10 citrulline, or in form of liposomes; together with one or more pharmaceutically acceptable excipients or carriers for the treatment of hair loss in a subject suffering from a hair loss disorder. A method for the treatment of a subject suffering or susceptible to suffer from a hair loss disorder, wherein the method comprises administering to said subject acetyl sh-hexapeptide-5 amide acetate, or in form of liposomes, for the treatment of hair loss in a subject suffering from a hair loss disorder, wherein said method comprises administering to a subject simultaneously, sequentially or separately tripeptide-10 citrulline, or in form of liposomes, and acetyl sh-hexapeptide-5 amide acetate, or in form of liposomes.

The fourth aspect of the invention refers to the cosmetic use of acetyl sh-hexapeptide-5 amide acetate, or in form of liposomes, or a cosmetic composition comprising a cosmetically effective amount of acetyl sh-hexapeptide-5 amide acetate together with one or more cosmetically acceptable excipients or carriers; as hair care agent in a subject not suffering from a hair loss disorder, wherein the hair care comprises ameliorating at least one of the following symptoms: hair brittleness, hair thinning, hair breakage, lack of hair elasticity, lack of hair brightness, lack of hair volume, hair roughness, hair flaking, hair dehydration, reduction of hair density and hair dryness. The term "cosmetically effective amount" refers to the amount of an active ingredient for instance acetyl sh-hexapeptide-5 amide acetate, or in form of liposomes, and/or tripeptide-10 citrulline , or in form of liposomesthat, when administered/applied, is sufficient to improve the appearance or to beautify, preserve, protect the hair in a subject not suffering from a hair loss disorder, ameliaorating at least one of the following symptoms: hair brittleness, hair thinning, hair breakage, lack of hair elasticity, lack of hair brightness, lack of hair volume, hair roughness, hair flaking, hair dehydration, reduction of hair density and hair dryness. Therefore, the above cosmetic use are adjectivally used for a non-medical application. The particular dose of the active ingredient for the cosmetic use according to the present invention will be determined by the particular circumstances surrounding the case, including the compound administered, the route of administration, the particular condition being treated, and the similar considerations.

The fifth aspect of the invention refers to the cosmetic use of acetyl sh-hexapeptide-5 amide acetate, or in form of liposomes, as hair care agent in a subject not suffering from a hair loss disorder; in combination therapy with tripeptide-10 citrulline, or in form of liposomes; wherein the hair care comprises ameliorating at least one of the following symptoms: hair brittleness, hair thinning, hair breakage, lack of hair elasticity, lack of hair brightness, lack of hair volume, hair roughness, hair flaking, hair dehydration, reduction of hair density and hair dryness. Again, it means that the cosmetic use involve the use of acetyl sh-hexapeptide-5 amide acetate and indications for the use of acetyl sh-hexapeptide-5 amide acetate, or in form of liposomes, in combined therapy with tripeptide-10 citrulline, or in form of liposomes.

The sixth aspect of the invention refers to the cosmetic use of acetyl sh-hexapeptide-5 amide acetate, or in form of liposomes; and tripeptide-10 citrulline, or in form of liposomes, as hair care agent in a subject not suffering from a hair loss disorder; wherein the hair care comprises ameliorating at least one of the following symptoms: hair brittleness, hair thinning, hair breakage, lack of hair elasticity, lack of hair brightness, lack of hair volume, hair roughness, hair flaking, hair dehydration, reduction of hair density and hair dryness.

In an embodiment, the cosmetic use of acetyl sh-hexapeptide-5 amide acetate, or in form of liposomes; and tripeptide-10 citrulline, or in form of liposomes, as hair care agent as defined above is one wherein the cosmetic treatment comprises administering to a subject:
either
(c) a cosmetic composition comprising:
   a cosmetically effective amount of acetyl sh-hexapeptide-5 amide acetate, or in form of liposomes; and
   a cosmetically effective amount of tripeptide-10 citrulline,or in form of liposomes;
   together with one or more cosmetically acceptable excipients or carriers;
   or alternatively,
(d) simultaneously, sequentially or separately:
   (d1) a cosmetic composition comprising a cosmetically effective amount of acetyl sh-hexapeptide-5 amide acetate, or in form of liposomes; together with one or more cosmetically acceptable excipients or carriers; and
   (d2) a cosmetic composition comprising a cosmetically effective amount of tripeptide-10 citrulline or in form of liposomes; together with one or more cosmetically acceptable excipients or carriers.

In an embodiment, the cosmetic use of acetyl sh-hexapeptide-5 amide acetate, or in form of liposomes; and tripeptide-10 citrulline, or in form of liposomes , as hair care agent as defined above is one wherein the cosmetic treatment comprises administering to a subject: (c) a cosmetic composition comprising: a cosmetically effective amount of acetyl sh-hexapeptide-5 amide acetate, or in form of liposomes; and a cosmetically effective amount of tripeptide-10 citrulline or in form of liposomes; together with one or more cosmetically acceptable excipients or carriers. Again, it means that the acetyl sh-hexapeptide-5 amide acetate and the tripeptide-10 citrulline are in a single unit dosage form as disclosed above for composition (a).

In an embodiment, the cosmetic use of acetyl sh-hexapeptide-5 amide acetate, or in form of liposomes; and tripeptide-10 citrulline, or in form of liposomes, as hair care agent as defined above is one wherein the cosmetic treatment comprises administering to a subject: (d) simultaneously, sequentially or separately:
(d1) a cosmetic composition comprising a cosmetically effective amount of acetyl sh-hexapeptide-5 amide acetate, or in form of liposomes; together with one or more cosmetically acceptable excipients or carriers; and
(d2) a cosmetic composition comprising a cosmetically effective amount of tripeptide-10 citrulline or in form of liposomes; together with one or more cosmetically acceptable excipients or carriers. Again, it means that the acetyl sh-hexapeptide-5 amide acetate, or in form of liposomes; and tripeptide-10 citrulline, or in form of liposomes, are in different single unit dosage forms which can be administered simultaneously, sequentially or separately as disclosed above for compositions (b).

In an embodiment, the acetyl sh-hexapeptide-5 amide acetate for use as defined in the first, second or third aspect of the invention; or alternatively the cosmetic use of acetyl sh-hexapeptide-5 amide acetate of the fourth, fifth and sixth aspect of the invention, is one wherein the treatment is administered once daily. The acetyl sh-hexapeptide-5 amide acetate for use as defined in the first, second or third aspect of the invention; or alternatively the cosmetic use of acetyl sh-hexapeptide-5 amide acetate of the fourth, fifth and sixth aspect of the invention, is one wherein the treatment is administered once daily at night. Commonly, the treatment is applied/administered once daily at night before going to bed.

The seventh aspect of the invention refers to a pharmaceutical or cosmetic composition comprising: a therapeutically or cosmetically effective amount of acetyl sh-hexapeptide-5 amide acetate, or in form of liposomes; and a therapeutically or cosmetically effective amount of tripeptide-10 citrulline, or in form of liposomes; together with one or more pharmaceutically or cosmetically acceptable excipients or carriers; and optionally one or more additional active ingredients.

For the purpose of the invention the term "pharmaceutical or cosmetic compositions" refers to all the compositions disclosed or claimed of the present invention, and particularly both the composition which comprises acetyl sh-hexapeptide-5 amide acetate and tripeptide-10 citrulline (cf. pharmaceutical composition (a) and cosmetic composition (c)) ; and also the compositions which comprises either acetyl sh-hexapeptide-5 amide acetate or alternatively the tripeptide-10 citrulline (cf. pharmaceutical composition (b1) and (b2); and cosmetic compositions (d1) and (d2)).

In an embodiment, the effective amount of acetyl sh-hexapeptide-5 amide acetate is from 0.001% to 5% by weight of the weight of the composition. In embodiment, the effective amount of tripeptide-10 citrulline is from 0.001% to 5% by weight of the weight of the composition. In an embodiment, the pharmaceutical or cosmetic compositions as defined above comprises from 0.001% to 5% by weight of the weight of the composition of acetyl sh-hexapeptide-5 amide acetate. In an embodiment, the pharmaceutical or cosmetic compositions as defined above comprises from 0.001% to 5% by weight of the weight of the composition of tripeptide-10 citrulline. In an embodiment, the pharmaceutical or cosmetic compositions as defined above comprises: from 0.001% to 5% by weight of the weight of the composition of acetyl sh-hexapeptide-5 amide acetate; and from 0.001% to 5% by weight of the weight of the composition of tripeptide-10 citrulline.

As it is mentioned above, the compositions as defined above comprises one or more pharmaceutically or cosmetically acceptable excipients or carriers. The term "pharmaceutically acceptable" refers to that excipients or carriers suitable for use in the pharmaceutical technology for preparing compositions with medical use. And the terms "cosmetically acceptable" or "dermatological acceptable" have the same meaning and which are herein used interchangeably. They refer to that excipients or carriers suitable for use in contact with human skin/scalp without undue toxicity, incompatibility, instability, allergic response, among others. The excipients or carriers used have affinity for the skin/scalp, are well tolerated, stable, and are used in an amount adequate to provide the desired consistency, and ease application. The appropriate excipients and/or carriers, and their amounts, can readily be determined by those skilled in the art according to the type of formulation being prepared to provide the desired consistency and ease application.

Examples of appropriate excipients or carriers can include, but not limited to, vehicle, dispersing agent, viscosity controlling, pH adjuster, preservative, antioxidant, surfactant, emulsifier and a mixture thereof

The term "vehicle" refers to a substance that facilitates the use of the active ingredients mixed with it. Examples of vehicles include, but are not limited to, water, glycols such as glycerin, propylene glycol, pentylene glycol and butylene glycol; alcohols such as ethanol and isopropanol; particularly water.

The term "preservative" refers to a material that prevents or reduces or slows down microbial growth, providing that the stability of the emulsion is not affected. Examples of appropriate preservative agents include, but are not limited to, benzoic acid, butylparaben, ethylparaben, propylparaben, methylparaben, sorbic acid, potassium sorbate, sodium benzoate, potassium sorbate, phenoxyethanol, triclosan, ethylhexylglycerin, dehydroacetic acid and a salt thereof, or their mixtures. If present, the amount of the preservatives in the compositions of the present invention is from 0.5 to 5% by weight.

The term "surfactant" refers to a material which lowers the surface tension of a liquid and the interfacial tension between two liquids, allowing their easier spreading. Surfactants have a hydrophilic head that is attracted to water molecules and a hydrophobic tail that repels water and simultaneously attaches itself to oil and grease in dirt. These opposing forces loosen the dirt and suspend it in the water, having the ability to remove it from surfaces such as the human skin, textiles, and other solids, when surfactants are dissolved in water. Examples of appropriate surfactant agents include, but are not limited to, non-ionic surfactants, cationic surfactants, amphoteric surfactants, zwitterionic surfactants, and mixtures thereof. Examples of specific surfactants appropriate for the present invention include, but are not limited to, polyglyceryl-4 diisostearate/ polyhydroxy stearate/sebacate, hydrogenated castor oil, PEG-40 hydrogenated castor oil, polyoxyethylene 2 stearyl ether, polyoxyethylene 20 stearyl ether, and mixture thereof. If present, the amount of surfactant in the compositions of the present invention is from 0.05% to 10% by weight.

The terms "emulsifying agent" or "emulsifying", which are used interchangeably herein, refer to any substance that reduces surface tension, promoting the formation of intimate mixtures of immiscible liquids by altering the interfacial tension. The emulsifier stabilizes an emulsion by increasing its kinetic stability. Examples of suitable emulsifiers include, but are not limited to, glyceryl trioleate, glyceryl oleate, sucrose acetylated distearate, sorbitan trioleate, polyoxyethylene monostearate, glycerol monooleate, sucrose distearate, polyethylene glycol monostearate (ethylene phenoxypoly ) ethanol, deacilerine penta-isostearate, sorbitan sesquioleate, hydroxylated lanolin, lecithin, lanolin, triglyceryl diisostearate, polyoxyethyleneleyl ether, sodium stearoyl 2-lactylate, sodium lauroyl lactylate, stearoyl sodium lactylate, sterocosaroyl lactylate cetearyl, methylglucoside sesquistearate, sorbitan monopalmitate, methoxy polyethylene glycol-22 / dodecyl glycol copolymer, polyethylene glycol-45 / dodecyl glycol copolymer, glyceryl stearate and polyethylene glycol 400 distearate / candelion glycol distearate salvado-3 esters of polygobacteria 400, cetyl phosphate, cetyl potassium phosphate, lecithin or their mixtures. If present, the amount of surfactant in the compositions of the present invention is from 0.05% to 5% by weight.

The terms "pH adjuster" and "pH regulating agent" and "buffer" and "buffering agent" have the same meaning and are used interchangeable. They refer to a substance that actively adjust and regulates the pH value of a solution to which they have been added. The commonly used pH regulating agents can include, but are not limited to, strong acids (i.e., acids that are completely dissociated in aqueous solution) and strong bases (i.e., bases that are completely dissociated in aqueous solution), acidic buffering agents and alkaline buffering agents, and nicotine. Examples of pH adjuster includes, but not limited to, sodium citrate, citric acid, dehydroacetic dcid or a salt thereof, lactic acid, sodium hydroxide, triethanolamine and mixture thereof. If present, the amount of pH regulating agent is from 0.01 to 5% by weight.

The term "thickening agent" or "thickener" or "viscosity agent" or "viscosity controlling" which is herein used interchangeably refers to a material that increases its viscosity without substantially modifying its other properties. Appropriate thickener includes, but are not limited to, natural and synthetic or semi-synthetic thickeners which derive from various sources and consist of very different molecular structures including polysaccharides, proteins, and alcohols. Examples of appropriate thickener include cetyl alcohol, stearyl alcohol, stearic acid, cellulose derivatives such as hydroxyethylcellulose, guar gum, locust bean gum, xanthan gum, gelatine, and polymeric compounds such as acrylic-based polymer (carbomer). If present, the amount of the viscosity agent is from 0.1 to 10% by weight.

The term "antioxidant" refers to any substance that delays or prevents the oxidation of any oxidizable component of the emulsion, such as for example fatty or oil components. Examples of antioxidants can include, but are not limited to, vitamin C, vitamin E such as tocopheryl acetate, vitamin A, niacinamide, butylated hydroxy-toluene (BHT), butylated hydroxyanisol (BHA), hydroxyacetophenone and mixture thereof. If present, the amount of antioxidants up to 0.1% by weight.

The term "dispersing agent" refers to any substance that promotes the formation and stabilization of a dispersion of one substance in another. Examples of dispersing agents may be a sugars and hydrophilic polymer includes, but not limited to maltodextrin and a mixture thereof. If present, the amount of dispersing agent is from 0.5% to 5% by weight.

As it is disclosed in the present application, the compositions as defined above can further comprise one or more additional pharmaceutically or cosmetically active ingredients. The presence of additional active ingredients and their amounts can readily be determined by those skilled in the art according to the type of formulation being prepared to provide the appropriate pharmaceutically or cosmetically effect. Examples of appropriate additional active ingredients can include, but are not limited to, hair conditioning agent, skin/scalp conditioning agent, anti-hair loss agent, sunscreens, anti-dandruff agent, soothing agent, anti-seborrhea agent, hair densifying agent and a mixture thereof.

The term "hair conditioning agent" active ingredient refers to any substance that is suitable for use in improving the condition of the hair giving them a soft and smooth appearance. Examples of appropriate hair conditioning agents include, but not limited to, one or more alkoxylated alcohols, alkoxylated amides, alkoxylated carboxylic acids, cationic surfactants, collagens, dimethicone polyols, esters (e.g., glyceryl esters), halogenated quaternary ammonium compounds, keratins, modified silicones, proteins, polymeric ethers such as panthenyl ethyl ether, quaternary ammonium compounds, or sorbitan derivatives, sugars such as inositol and lactose, milk or milk derived compounds such as milk protein, and mixtures thereof. If present, the amount of hair conditioning agent is from 0.1 to 5% by weight.

The term "skin/scalp conditioning agent" refers to a substance that increase the water content of the top layers of the skin/scalp and/or give the skin/scalp a soft and smooth appearance. Examples of appropriate skin/scalp conditioning agents can include, but are not limited to, esters of glycerine and C₁₅-C₂₅ fatty acid such as glyceryl oleate; C₁₅-C₂₅ fatty alcohol such as lauryl alcohol, myristyl alcohol, palmityl alcohol, stearyl alcohol, linoleyl alcohol, and oleyl alcohol; diols such as propyleneglycol, propanediol, 1,2-hexanediol, ethylhexylglycerine, glycerol, caprylyl glycol, propylene glycol, butylene glycol, pentylene glycol, hexylene glycol, and dipropylene glycol or diethylene glycol. If present, the amount of skin/scalp conditioning agent is from 0.01 to 5% by weight.

The term "anti-dandruff agent" refers to any substance that act against the appearance of dandruff. Dandruff is a skin disorder characterized by massive peeling of the stratum corneum. It is a common condition in men and women of all ages, which occurs equally in both sexes and often causes skin irritation and discomfort to those who suffer it. Examples of antidandruff agents suitable for the present invention include, without limitation, cytostatic agents that decrease the rate of cell division such as zinc pyrithione and selenium disulfide; antimicrobial agents with action against Malasezzia furfur such as pyroctone olamine and essential oil of tea tree.

The term "anti-hair loss agent" refers to any substance that act against hair loss, which occurs when hair loss causes a decrease in the hair population (capillary density). Examples of suitable anti-hair loss agents for the present invention include, without limitation, blood flow stimulating agents such as minoxidiland derivatives, menthol, caffeine (particularly maltodrextrin encapsulated caffeine) and Panax ginseng root extract; nutritional agents such as sulfur amino acids and derivative thereof (acetyl cysteine and acetyl methionine), and minerals such as zinc; protein hydrolysates; vitamins (vitamin E and salts and derivative thereof; vitamin C and salts and derivative thereof, vitamin of B group such as B5, B6 and B8); 5-alpha-reductase enzyme inhibitors such as plant extracts of saw palmetto and hops; antioxidants such as Malus Domestica Fruit Cell Culture Extract and a mixture thereof. If present, the amount of additional pharmaceutically or cosmetically active ingredients is from 1 to 45%.

The term "soothing agent" refers to any substance that act to decrease irritation or inflammation, itching and discomfort of the sensitive scalp. The scalp, being a highly vascularized and innervated area, is more sensitive than the skin of the rest of the body. When the scalp is irritated, the vascularity is altered and has a direct impact on the health of the hair. The horny layer of the scalp is more disorganized than other areas of the body; Due to this, cell renewal is faster and there is a greater sensitization of the scalp. In some situations, it shows exaggerated sensitivity to some external or internal stimuli, and responds with redness, itching and flaking. Examples of suitable soothing agents for the present invention include, without limitation, irritation calming ingredients such as bisabolol, aloe, calendula and chamomile plant extracts; ingredients that prevent flaking and hydrate the scalp such as glycerin and panthenol; nourishing agents such as phospholipids, ceramides, and vegetable oils; and mixtures thereof.

The terms "anti-grease agent" or "anti-seborrhea agent" have the same meaning and are used interchangeably. They refer to any substance that act by reducing excess sebaceous secretion. Seborrhea is an alteration in the amount and composition of the sebum in the sebaceous glands. Examples of suitable anti-seborrhea agents for the present invention include, without limitation, ingredients that control excess fat secretion such as 5-alpha-reductase enzyme inhibitors such as saw palmetto, pumpkin and hop plant extracts; sulfur derivatives such as sulfur amino acids; antioxidant ingredients that prevent rancidity of excess sebum such as tocopherol acetate; astringent ingredients that reduce pore size such as zinc salts; and mixtures thereof.

The term "sunscreen" refers to any substance capable of reducing or stopping the UV radiation, particularly UVA radiation, UVB radiation or both. Examples of suitable sunscreen for the present invention include amoung others, without limitation, diethylamino hydroxybenzoyl hexyl benzoate, disodium phenyl dibenzimidazole tetrasulfonate, octyl methoxycinnamate, butyl methoxydibenzoylmethane, oxybenzone, sulibenzone, terephthalylidene dicamphor sulfonic acid and a mixture thereof.

The terms "hair densifying agent" and "hair densifier" have the same meaining and are used interchangeable. They refer to any substance capable of providing volume and shape to the hair. Examples of suitable densifiers for the present invention include, without limitation, castor oil and derivative thereof, Ginseng extract, vitamins such as B5, C, E and derivatives thereof, natural oils such as sunflower, almond or coconut oil, and a mixture thereof.

The compositions of the present invention may contain other ingredients, such as fragrances, colorants and other components known in the state of the art.

In an embodiment, the compositions as defined above are topical pharmaceutical or cosmetic compositions comprising one or more appropriate topical pharmaceutically or cosmetically acceptable excipients or carriers. In an embodiment, the topical compositions of the invention can be formulated in several forms that include, but are not limited to, solution, emulsion, surfactant base, suspension, and gel. In an embodiment, the compositions of the present application are commercially available as cream, lotion, ointment, salve, solution, mousse, foams, sprays, paste, shampoo, conditioner, mask, tonics, serums, tablets, capsules, and dyes.

In one embodiment, the topical compositions are used is formulated as an emulsion. The term "emulsion" refers to a dispersed system comprising at least two immiscible phases, one phase dispersed in the other in the form of drops. The emulsifying agents mentioned above are included to improve stability. When the dispersed phase is water and oil is the dispersion medium, the emulsion is called a water-in-oil (w / o) emulsion. On the contrary, when it is the oil that is dispersed as drops in the aqueous phase, the emulsion is called oil in water (o / w). Other types of emulsions known in the state of the art are multiple emulsions, such as water-in-oil-in-water (w / o / w) emulsions, GELTRAP emulsions, where the internal aqueous phase is gelled and is covered by the oil phase, and SWOP emulsions, also known as inversion emulsions. Preferably, the compositions of the present application are topical emulsion compositions. In fact, the compositions that are in form of emulsions for use in the present invention are compatible with creams and lotions.

In one embodiment, the topical composition used is formulated as a surfactant base. The terms "surfactant base" or "surfactant system", which are used interchangeably herein, refer to a mixture of at least two surfactants, preferably anionic or amphoteric surfactants, which tends to form spherical micelles that are isotropic with low viscosity , or they tend to form crystalline liquid phases of lamellar and hexagonal phases, which are anisotropic with higher viscosity. In fact, the compositions that are in form of surfactant bases for use in the present invention are compatible with rinse-off products (also called rinse-off) such as shampoos, shower gels, conditioner, mask and dyes.

In one embodiment, the topical composition used is formulated as a solution. The term "solution" refers to a chemically and physically homogeneous mixture of two or more substances. The solution can comprise a solid dispersed in a liquid, solid or semi-solid medium. In fact, the compositions that are in form of surfactant bases for use in the present invention are compatible with leave-on products (also called leave-on) such as the hair lotions.

In an embodiment, the compositions as defined above, particularly topical compositions, and below comprises the following excipients:
from 0.5 to 5 % by weight of dispersing agent;
from 0.05 to 5% by weight of emulsifier;
from 0.05 to 10 % by weight of viscositity controlling;
from 0.5 to 5 % by weight of preservative;
from up to 0.1% by weight of antioxidant;
from 0.01 to 5% by weight of pH adjuster agent; and
from 70 to 90% of solvents;
being the sum of all the components of the composition up to 100% by weight.

In an embodiment, the compositions as defined above, particularly topical compositions, and below comprises:
from 0.001 to 5% by weight of acetyl sh-hexapeptide-5 amide acetate;
from 0.5 to 5 % by weight of dispersing agent;
from 0.05 to 5% by weight of emulsifier;
from 0.05 to 10 % by weight of viscositity controlling;
from 0.5 to 5 % by weight of preservative;
from up to 0.1% by weight of antioxidant;
from 0.01 to 5% by weight of pH adjuster agent; and
from 70 to 90% of solvents;
optionally, one or more additional active ingredients;
being the sum of all the components of the composition up to 100% by weight.

In an embodiment, the compositions as defined above, particularly topical compositions, and below comprises:
from 0.001 to 5 % by weight of acetyl sh-hexapeptide-5 amide acetate in form of liposomes, particularly lecithin liposomes;
from 0.5 to 5 % by weight of dispersing agent;
from 0.05 to 5% by weight of emulsifier;
from 0.05 to 10 % by weight of viscositity controlling;
from 0.5 to 5 % by weight of preservative;
from up to 0.1% by weight of antioxidant;
from 0.01 to 5% by weight of pH adjuster agent; and
from 70 to 90% of solvents;
optionally, one or more additional active ingredients;
being the sum of all the components of the composition up to 100% by weight.

In an embodiment, the compositions as defined above, particularly topical compositions, and below comprises:
from 0.001 to 5% by weight of tripeptide-10 citrulline;
from 0.5 to 5 % by weight of dispersing agent;
from 0.05 to 5% by weight of emulsifier;
from 0.05 to 10 % by weight of viscositity controlling;
from 0.5 to 5 % by weight of preservative;
from up to 0.1% by weight of antioxidant;
from 0.01 to 5% by weight of pH adjuster agent; and
from 70 to 90% of solvents;
optionally, one or more additional active ingredients;
being the sum of all the components of the composition up to 100% by weight.

In an embodiment, the compositions as defined above, particularly topical compositions, and below comprises:
from 0.001 to 5 % by weight of tripeptide-10 citrulline in form of liposomes, particularly lecithin liposomes;
from 0.5 to 5 % by weight of dispersing agent;
from 0.05 to 5% by weight of emulsifier;
from 0.05 to 10 % by weight of viscositity controlling;
from 0.5 to 5 % by weight of preservative;
from up to 0.1% by weight of antioxidant;
from 0.01 to 5% by weight of pH adjuster agent; and
from 70 to 90% of solvents;
optionally, one or more additional active ingredients;
being the sum of all the components of the composition up to 100% by weight.

In an embodiment, the compositions as defined above, particularly topical compositions, and below comprises:
from 0.001 to 5 % by weight of acetyl sh-hexapeptide-5 amide acetate;
from 0.001 to 5 % by weight of tripeptide-10 citrulline:
from 0.5 to 5 % by weight of dispersing agent;
from 0.05 to 5% by weight of emulsifier;
from 0.05 to 10 % by weight of viscositity controlling;
from 0.5 to 5 % by weight of preservative;
from up to 0.1% by weight of antioxidant;
from 0.01 to 5% by weight of pH adjuster agent; and
from 70 to 90% of solvents;
optionally, one or more additional active ingredients;
being the sum of all the components of the composition up to 100% by weight.

In an embodiment, the compositions as defined above, particularly topical compositions, and below comprises:
from 0.001 to 5 % by weight of acetyl sh-hexapeptide-5 amide acetate in form of liposomes, particularly lecithin liposomes;
from 0.001 to 5 % by weight of tripeptide-10 citrulline in form of liposomes, particularly lecithin liposomes;
from 0.5 to 5 % by weight of dispersing agent;
from 0.05 to 5% by weight of emulsifier;
from 0.05 to 10 % by weight of viscositity controlling;
from 0.5 to 5 % by weight of preservative;
from up to 0.1% by weight of antioxidant;
from 0.01 to 5% by weight of pH adjuster agent; and
from 70 to 90% of solvents;
optionally, one or more additional active ingredients;
being the sum of all the components of the composition up to 100% by weight.

It is also a part of the invention a process for the preparation of the compositions as defined herein above and below. They can readily be determined by those skilled in the art from those processes disclosed in the state of the art according to the type of formulation being prepared. Typically, the process for the preparation of suspensions or solutions comprises mixing sequentially each one of the ingredients separately under agitation under obtaining the final suspension or solution. In an embodiment, when the composition comprises liposomes of acetyl sh-hexapeptide-5 amide acetate and/or tripeptide-10 citrulline, then the process previously comprises preparing the liposome in an aqueous solution by mixing the acetyl sh-hexapeptide-5 amide acetate or the tripeptide-10 citrullin with the phospholips (such as lecithin) and subsequently add the reamining of components as mentioned above. In an embodiment, when the composition comprises extracts of natural products such as Malus Domestica fruit Extract and Panax root extract, the process further comprises an additional step that comprises preparing an aqueous suspension of the extracts, and the subsequent addition to the mixture that comprises all the components except from the extract. In case emulsions, typically, the process for the preparation of emulsions comprises: (i) firstly, preparing the aqueous phase(s) and the oil phase separately; and (ii) secondly, mixing both phases until obtaining the water-in-oil-emulsion as it is shown in the experimental section.

The compositions as defined herein above and below can be defined by its preparation process. Thus, a composition as defined herein below or above obtainable by the process as defined above is also part of the invention. For the purposes of the invention the expressions "obtainable", "obtained" and equivalent expressions are used interchangeably, and in any case, the expression "obtainable" encompasses the expression "obtained". All the embodiments disclosed above for the process of the invention applies also for composition obtainable by this process.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention.

### Examples

### 1. Compositions of acetyl sh-hexapeptide-5 amide acetate and tripeptide-10 citrulline

### 1.1. Compositions

These examples illustrate topical liquid compositions (Examples 1 and 2) in accordance with the present invention. Composition 1 (Example 1) comprises lecithin liposomes of acetyl sh-hexapeptide-5 amide acetate and lecithin liposomes of tripeptide-10 citrulline; and composition 2 (Example 2) comprises acetyl sh-hexapeptide-5 amide acetate and lecithin liposomes of tripeptide-10 citrulline.

Table 1 shows the qualitive and quantitative formulation, wherein the amount of the comercial product used for the preparation of the compositions of the invention and also the amount of the ingredient contained in the comercial product both expressed in % by weight in relation to the total weight of the composition.

**Table 1**

| **Composition 1 (Example 1)** | | | | | |
|---|---|---|---|---|---|
| **Commercial product** | **Ingredient** | | **Function** | **Amount (%)** | |
| | | | | **Commercial Product** | **Ingredient** |
| Encapsulated caffeine | Caffeine | | Additional active ingredient | 2% | 0.96% |
| - | **Lecithin liposomes of acetyl sh-hexapeptide-5 amide acetate ^{(a)}** | lecithin | **Active ingredient** | 2% | 0.6% |
| Versillin^{®} | | acetyl sh-hexapeptide-5 amide acetate | | **3%** | **0.0015%^{(b)}** |
| Decorinyl^{®} | **Lecithin liposomes of tripeptide-10 citrulline** | lecithin | **Active ingredient** | **3%** | **0.006%^{(c)}** |
| | | tripeptide-10 citrulline | | | |
| Propylene glycol | Propylene glycol | | Solvent | - | 1% |
| water sol. stem cells from apple, | Malus Domestica fruit cell Culture Extract | | Additional active ingredient | 1% | 0.99% |
| Cremophor CO40 | PEG-40 hydrogenated castor oil | | surfactant | 2% | 2% |
| Water sol. Ginseng extract | Panax root extract | | Additional active ingredient | 1% | 0.05% |
| water solution of sulfur amino acids | Acetyl cysteine | | Additional active ingredient | 1.5% | 0.001875% |
| | Acetyl methionine | | | | 0.001875% |
| water | Water | | Solvent | - | q.s.f. 100% |

| | | | | | |
|---|---|---|---|---|---|
| (a) the lecithin liposomes of acetyl sh-hexapeptide-5 amide acetate were prepared in-situ before the preparation of the composition by mixing commercially available lecithin and acetyl sh-hexapeptide-5 amide acetate (Versillin^{®}) as disclosed below. (b) After the preparation of the lecithin liposomes of acetyl sh-hexapeptide-5 amide acetate, the composition 1 contains 0.0015% by weight of acetyl sh-hexapeptide-5 amide acetate. (c) Composition 1 contains 0.006% by weight of tripeptide-10 citrulline provided from the commercial lecithin liposome tripeptide-10 citrulline. | | | | | |

**Table 1-cont**

| **Composition 2 (Example 2)** | | | | | |
|---|---|---|---|---|---|
| **Commercial product** | **Ingredient** | | **Function** | **Amount (%)** | |
| | | | | **Commercial product** | **Ingredient** |
| Encapsulated caffeine | Caffeine | | Additional active ingredient | 2% | 0.96% |
| Versillin^{®} | **acetyl sh-hexapeptide-5 amide acetate** | | **Active ingredient** | **3%** | **0.0015%** |
| Decorinyl^{®} | **Lecithin liposomes of tripeptide-10 citrulline** | lecithin | **Active ingredient** | **3%** | **0.006%^{(d)}** |
| | | tripeptide-10 citrulline | | | |
| Propylene glycol | Propylene glycol | | Solvent | - | 1% |
| Water sol. stem cells apple | Malus Domestica fruit cell Culture Extract | | Additional active ingredient | 1% | 0.99% |
| Cremophor CO40 | PEG-40 hydrogenated castor oil | | surfactant | 2% | 2% |
| Water sol. Ginseng extract | Panax root extract | | Additional active ingredient | 1% | 0.05% |
| water solution of sulfur amino acids | Acetyl cysteine | | Additional active ingredient | 1.5% | 0.001875% |
| | Acetyl methionine | | | | 0.001875% |
| water | Water | | Solvent | - | q.s.f. 100% |

| | | | | | |
|---|---|---|---|---|---|
| (d) Composition 2 contains 0.006% by weight of tripeptide-10 citrulline provided from the commercial lecithin liposome tripeptide-10 citrulline. | | | | | |

### 1.2. Preparation process

The compositions of the present invention (Examples 1-2) were prepared following the processes disclosed herein below.

### Composition 1 (Example 1)

1. Preparation of a suspension of liposomes of acetyl sh-hexapeptide-5 amide acetate. Mixing 400mg of lecithin commercially available with 1.5mg of acetyl sh-hexapeptide-5 amide acetate (Versillin) at room temperature for 20 min. The liposomes thus obtained were suspended in water (100ml) under agitation to obtain suspension 1.
2. Preparation of the suspension of extracts of Malus Domestica fruit cell Culture and Panax root. The amount of apple extract and the ginseng extract disclosed in Table 1 were solubilised with PEG-40 Hydrogenated Castor Oil under agitation.
3. Preparation of the final composition. Propylene glycol and phenoxyethanol were added under agitation to suspension 1 previously prepared in step 1. The rest of ingredients except from the suspension of extracts was sequentially added to the suspension previously obtained; and finally, the extract suspension obtained in step 2 was added to the previous resulting mixture under agitation to obtain the composition of Example 1.

### Composition 2 (Example 2)

Composition 2 was prepared following the process as defined above for the composition 1, but without the preparation of the liposomes of acetyl sh-hexapeptide-5 amide acetate (step 1). Meanwhile, acetyl sh-hexapeptide-5 amide acetate and the mixture obtained is diluted in an aqueous medium.

### 2. Effectivity Test

### 2.1. In vitro assessment in Dermal Papilla cells

This assay determined the beneficial effect of the acetyl sh-hexapeptide-5 amide acetate in the reduction of the hair loss in an in vitro assay, using Dermal Papilla cells and analysed through gene expression analysis. In particular, this test determined the effect of the acetyl sh-hexapeptide-5 amide acetate alone or in combination with tripeptide-10 citrulline after 24 hours of treatment of Human Follicle Dermal Papilla Cells (HFDPC), and through analysis of Fibroblast Growth Factor-7 (FGF7), Insulin-like Growth Factor-1 (IGF1), and Marker of Proliferation KI-67 (MKI67) gene expression, using Quantitative reverse transcription PCR (RT-qPCR) method.

Analytical equipment: Microscope, incubator, refrigerated centrifuge, statistical analysis software, micropipettes, pipettes, propipette, rack, quantifier Nano-Drop spectrophotometer, laminar flow hood, QuantStudio 5 (Applied Biosystem) Quantitative real-time PCR, vortex, Bürker chamber, heating block, thermocycler, and consumables.

Reagents: Distilled water (Braun), HFDPC culture medium (Promocell), nutrient solution mix (Promocell), Phosphate buffered saline (Sigma), Trypan Blue Solution (Bio-Rad), Ethanol (Sigma-Aldrich), DMSO (Merck), Trypsin (Sigma), DNAse-I (Qiagen), RNeasy extraction kit (Qiagen), PrimeScript RT reagent kit (Perfect Real Time- Takara), oligonucleotides for RT-PCR amplification of FGF7, IGF1, MKI67 and ACT, SYBR ^{®} master mix, liquid nitrogen.

### Samples:

- Sample 1: Aqueous solution comprising 0.0015% of acetyl sh-hexapeptide-5 amide acetate.
- Sample 2: Aqueous solution comprising 0.0015% by weight of acetyl sh-hexapeptide-5 amide acetate and 0.006% by weight of tripeptide-10 citrulline

Methodology: Cell number and viability were determined using a Bürker chamber and Trypan-Blue staining. For the gene expression assay, HFDPC cells (Human Follicle Dermal Papilla Cells) (commercially available and isolated from human dermis of the scalp, originating from the occipital or temple region) were seeded at a 200.000 cells/well in a 6-well plate with complete growth medium. 24 hours later, it was replaced by new culture medium supplemented with samples 1 and 2 separately at 1 % and 5 %. The untreated control samples were maintained in culture medium.

After 24 hours of incubation, the medium was removed, and cells were washed with PBS (phosphate buffered saline). Cells were then collected in lysis buffer to proceed with RNA extraction. Total RNA was extracted using RNeasy^{®} mini kit (Qiagen) and treated with DNase-I to remove any contamination with genomic DNA. RNA quality and quantity were assessed in a Nano-Drop spectrophotometer, and 500 ng of total RNA were used to synthesize cDNA (cf. protocol of Purification of total RNA from Animal cells using spin technology of the RNEasy^{®} Mini Handbook 10/2019, pp. 27-34; including the on-column DNase digestion with the RNAse-Free DNase set (appendix D) of the RNEasy^{®} Mini Handbook 10/2019, pp. 82-84. The suitability of each primer pair used in this study for RT-qPCR (ACT, FGF7, IGF1 and MKI67), was previously evaluated to determine melting curves, efficiency of amplification and primer specificity. Finally, quantitative PCR was performed in a real time PCR machine (QuantStudio 5 Applied Biosystem- LightCycler/LightCycler 480 System) following the process as disclosed in the RNEasy^{®} Mini Handbook 10/2019, pp. 8 of the addapted by preparing 10 microliters and following the next steps: Denature 95°C 2 min (Ramp rate: 4.4°C/sec) 1 cycle PCR Analysis Mode: Quantification 95°C 5 sec (Ramp rate: 4.4°C/sec) 62.5°C 7 sec (Ramp rate: 2.2°C/sec, Acquisition Mode: Single) 40 cycles Melting Analysis Mode: Melting Curves 95°C 5 sec (Ramp rate: 4.4°C/sec) 60°C 1 min (Ramp rate: 2.2°C/sec) 95°C (Ramp rate: 0.11°C/sec, Acquisition Mode: Continuous, Acquisitions: 5 per °C). One biological replicate with 4 technical replicates per condition was performed. All data were statistically analysed.

**Results:** The determination of FGF7, IGF1 and MKI56 gene expression is summarised herein below.

Fibroblast Growth Factor-7 (FGF7) gene expression: The results showed that Fibroblast Growth Factor-7 (FGF7) expression was significantly increased by 72.3 ± 12.5 % and 108.0 ± 8.3 % after treatment with acetyl sh-hexapeptide-5 amide acetate (sample 1), at 1 % and 5 %, respectively compared to the untreated control. Furthermore, the treatment with a combination of acetyl sh-hexapeptide-5 amide acetate and tripeptide-10 citrulline (sample 2), at 1 % and 5 % significantly increased Fibroblast Growth Factor-7 (FGF7) expression by 74.5 ± 7.2 %, and 156.2 ± 27.0 % respectively, compared to the untreated control.

Insulin-like Growth Factor-1 (IGF1) gene expression: The results showed that the treatment with acetyl sh-hexapeptide-5 amide acetate (sample 1) at 1 % and 5 % significantly increased IGF1 gene expression by 286.3 ± 72.0 and 300.2 ± 45.0 % respectively, compared to the untreated control. In the same way, the combination of acetyl sh-hexapeptide-5 amide acetate and tripeptide-10 citrulline (sample 2) at 5 % induced a statistically significant boost in the expression of this gene by 408.9 ± 62.5 %, compared to the untreated control.

Marker of Proliferation KI-67 (MKI67) gene expression: The results showed that the treatment with acetyl sh-hexapeptide-5 amide acetate (sample 1) at 5 %, significantly increased MKI67 gene expression by 100.4 ± 25.2 %, compared to the untreated control. Furthermore, a combination of acetyl sh-hexapeptide-5 amide acetate and tripeptide-10 citrulline (sample 2) at 1 % and 5% by weight, significantly increased MKI67 gene expression by 107.1 ± 14.5 % and 86.8 ± 19.2 % respectively %, compared to the untreated control.

### Conclusion

From the above-disclosed results, it is demonstrated that acetyl sh-hexapeptide-5 amide acetate, alone or in combination with tripeptide-10 citrulline, shows a stimulating effect on Human Follicle Dermal Papilla Cells (HFDPC), through a significant increase in the expression of Fibroblast Growth Factor-7 (FGF7), Insulin-like Growth Factor 1 (IGF1), and Ki67 (MKI67) genes. It is also observed that this stimulant effect enhancing the gene expression of FGF7 and IGF1 is higher at high concentration of acetyl sh-hexapeptide-5 amide acetate.

As the gene expression of Fibroblast Growth Factor-7 (FGF7), Insulin-like Growth Factor 1 (IGF1), and Ki67 (MKI67) has been previously correlated with cell proliferation and migration during development of hair follices, and therefore, in the promotion of hair follice growth; the above mentioned results allows demonstrating that acetyl sh-hexapeptide-5 amide acetate, alone or in combination with tripeptide-10 citrulline, is useful for the treatment of hair loss in a subject suffering from a hair loss disorder; and also appropriate for a cosmetic use in a subject not suffering a hair loss disorder for ameliorating at least one of the following symptoms: hair brittleness, hair thinning, hair breakage, lack of hair elasticity, lack of hair brightness, lack of hair volume, hair roughness, hair flaking, hair dehydration, reduction of hair density and hair dryness.

### Citation List

1. Protocol of Purification of total RNA from Animal cells using spin technology of the RNEasy^{®} Mini Handbook 10/2019, pp. 27-34.
2. Appendex D: On-column DNase digestion with the RNAse-Free DNase set of the RNEasy® Mini Handbook 10/2019, pp. 82-84.
3. RNEasy^{®} Mini Handbook 10/2019, pp. 8
4. "Adenosine stimulates fibroblast growth factor-7 gene expression via adenosine A2b receptor signaling in dermal papilla cells", Journal of Investigative Dermatology 2007, Jun ;127(6) :1318-25.

## Claims

1. Acetyl sh-hexapeptide-5 amide acetate, or in form of liposomes;
or a pharmaceutical composition comprising the therapeutically effective amount of acetyl sh-hexapeptide-5 amide acetate, or in form of liposomes together with one or more pharmaceutically acceptable excipients or carriers; for use in the treatment of hair loss in a subject suffering from a hair loss disorder.

2. Acetyl sh-hexapeptide-5 amide acetate, or in form of liposomes, for use in the treatment of hair loss in a subject suffering from a hair loss disorder, wherein the acetyl sh-hexapeptide-5 amide acetate, or in form of liposomes, is for use in combination therapy with tripeptide-10 citrulline, or in form of liposomes.

3. Acetyl sh-hexapeptide-5 amide acetate, or in form of liposomes; and tripeptide-10 citrulline, or in form of liposomes, for use in the treatment of hair loss in a subject suffering from a hair loss disorder.

4. The acetyl sh-hexapeptide-5 amide acetate, or in form of liposomes; and tripeptide-10 citrulline, or in form of liposomes, for use according to claim 3, wherein the treatment comprises administering to a subject:
either
(a) a pharmaceutical composition comprising:
a therapeutically effective amount of acetyl sh-hexapeptide-5 amide acetate, or in form of liposomes; and
a therapeutically effective amount of tripeptide-10 citrulline, or in form of liposomes;
together with one or more pharmaceutically acceptable excipients or carriers;
or alternatively,
(b) simultaneously, sequentially or separately:
(b1) a pharmaceutical composition comprising a therapeutically effective amount of acetyl sh-hexapeptide-5 amide acetate, or in form of liposomes; together with one or more pharmaceutically acceptable excipients or carriers; and
(b2) a pharmaceutical composition comprising a therapeutically effective amount of tripeptide-10 citrulline or in form of liposomes; together with one or more pharmaceutically acceptable excipients or carriers.

5. The acetyl sh-hexapeptide-5 amide acetate, or in form of liposomes, or the pharmaceutical composition for use according to any of the claims 1-4 in the treatment of hair loss wherein the treatment is carried out by re-growing of the hair, by prevention of further hair loss, by diminishing the rate of hair loss, by reducing the telogen phase and increasing the anagen phase and/or by enzimatic inhibition of 5-alpha reductase enzime.

6. Cosmetic use of acetyl sh-hexapeptide-5 amide acetate, or in form of liposomes; or of a cosmetic compositior comprising a cosmetically effective amount of acetyl sh-hexapeptide-5 amide acetate, or in form of liposomes together with one or more cosmetically acceptable excipients or carriers; as hair care agent in a subject not suffering from a hair loss disorder, wherein the hair care comprises ameliorating at least one of the following symptoms: hair brittleness, hair thinning, hair breakage, lack of hair elasticity, lack of hair brightness, lack of hair volume, hair roughness, hair flaking, hair dehydration, reduction of hair density and hair dryness.

7. Cosmetic use of acetyl sh-hexapeptide-5 amide acetate, or in form of liposomes, as hair care agent in a subject not suffering from a hair loss disorder; in combination therapy with tripeptide-10 citrulline, or in form of liposomes; wherein the hair care comprises ameliorating at least one of the following symptoms: hair brittleness, hair thinning, hair breakage, lack of hair elasticity, lack of hair brightness, lack of hair volume, hair roughness, hair flaking, hair dehydration, reduction of hair density and hair dryness.

8. Cosmetic use of acetyl sh-hexapeptide-5 amide acetate, or in form of liposomes; and tripeptide-10 citrulline, or in form of liposomes, as hair care agent in a subject not suffering from a hair loss disorder; wherein the hair care comprises ameliorating at least one of the following symptoms: hair brittleness, hair thinning, hair breakage, lack of hair elasticity, lack of hair brightness, lack of hair volume, hair roughness, hair flaking, hair dehydration, reduction of hair density and hair dryness.

9. The cosmetic use according to claim 8, wherein the cosmetic treatment comprises administering to a subject:
either
(c) a cosmetic composition comprising:
a cosmetically effective amount of acetyl sh-hexapeptide-5 amide acetate, or in form of liposomes; and
a cosmetically effective amount of tripeptide-10 citrulline or in form of liposomes;
together with one or more cosmetically acceptable excipients or carriers;
or alternatively,
(d) simultaneously, sequentially or separately:
(d1) a cosmetic composition comprising a cosmetically effective amount of acetyl sh-hexapeptide-5 amide acetate, or in form of liposomes; together with one or more cosmetically acceptable excipients or carriers; and
(d2) a cosmetic composition comprising a cosmetically effective amount of tripeptide-10 citrulline or in form of liposomes; together with one or more cosmetically acceptable excipients or carriers.

10. The acetyl sh-hexapeptide-5 amide acetate for use according to any of the claims 1-5: or alternatively, the cosmetic use according to any of the claims 6-9, wherein the treatment is administered once daily.

11. A pharmaceutical or cosmetic composition comprising:
a therapeutically or cosmetically effective amount of acetyl sh-hexapeptide-5 amide acetate, or in form of liposomes; and
a therapeutically or cosmetically effective amount of tripeptide-10 citrulline, or in form of liposomes;
together with one or more pharmaceutically or cosmetically acceptable excipients or carriers.

12. The acetyl sh-hexapeptide-5 amide acetate for use according to any of the claims 1-5: or alternatively, the cosmetic use according to any of the claims 6-10; or alternatively, the pharmaceutical or cosmetic composition according to claim 11; wherein the pharmaceutical and cosmetic composition is a topical composition.

13. The acetyl sh-hexapeptide-5 amide acetate for use according to any of the claims 1-5 and 12; or alternatively, the cosmetic use according to any of the claims 6-10 and 12; or alternatively, the pharmaceutical or cosmetic composition according to any of the claims 11 or 12; wherein the acetyl sh-hexapeptide-5 amide acetate is in form of liposomes and the tripeptide-10 citrulline is in form of liposomes; preferably lecithin liposomes.

14. The acetyl sh-hexapeptide-5 amide acetate for use according to any of the claims 1-5 and 12-13; or alternatively, the cosmetic use according to any of the claims 6-10 and 12-13; or alternatively, the pharmaceutical or cosmetic composition according to any of the claims 11-13; wherein:
the one or more pharmaceutically or cosmetically acceptable excipients or carriers are selected from the group consisting of vehicle, dispersing agent, viscosity controlling, pH adjuster, preservative, antioxidant, surfactant, emulsifier and a mixture thereof; and
the pharmaceutical or cosmetic composition further comprises one or more additional pharmaceutical or cosmetic active ingredients; particularly selected from the group consisting of hair conditioning agent, skin/scalp conditioning agent, anti-hair loss agent, sunscreens, anti-dandruff agent, soothing agent, anti-seborrhea agent, hair densifying agent and a mixture thereof.

15. The acetyl sh-hexapeptide-5 amide acetate for use according to any of the claims 1-5 and 12-14; or alternatively, the cosmetic use according to any of the claims 6-10 and 12-14; or alternatively, the pharmaceutical or cosmetic composition according to any of the claims 11-14; wherein: the effective amount of acetyl sh-hexapeptide-5 amide acetate is from 0.001% to 5% by weight; and/or the effective amount of tripeptide-10 citrulline is from 0.001% to 5% by weight.

## Patentansprüche

1. Acetyl-sh-Hexapeptid-5-amidacetat, oder in Form von Liposomen;
oder eine pharmazeutische Zusammensetzung, umfassend die therapeutisch wirksame Menge an Acetyl-sh-hexapeptid-5-amidacetat, oder in Form von Liposomen zusammen mit einem oder mehreren pharmazeutisch akzeptablen Hilfsstoffen oder Trägersubstanzen; zur Verwendung bei der Behandlung von Haarausfall bei einem Patienten, der an einer Haarausfallstörung leidet.

2. Acetyl-sh-hexapeptid-5-amidacetat, oder in Form von Liposomen, zur Verwendung bei der Behandlung von Haarausfall bei einem Patienten, der an einer Haarausfallstörung leidet, wobei das Acetyl-sh-hexapeptid-5-amidacetat, oder in Form von Liposomen, zur Verwendung in der Kombinationstherapie mit Tripeptid-10-Citrullin, oder in Form von Liposomen, bestimmt ist.

3. Acetyl-sh-hexapeptid-5-amidacetat, oder in Form von Liposomen; und Tripeptid-10-Citrullin, oder in Form von Liposomen, zur Verwendung bei der Behandlung von Haarausfall bei einem Patienten, der an einer Haarausfallstörung leidet.

4. Das Acetyl-sh-hexapeptid-5-amidacetat, oder in Form von Liposomen; und das Tripeptid-10-citrullin, oder in Form von Liposomen, zur Verwendung nach Anspruch 3, wobei die Behandlung die Verabreichung an einen Patienten umfasst von:
entweder
(a) einer pharmazeutische Zusammensetzung umfassend:
eine therapeutisch wirksame Menge an Acetyl-sh-Hexapeptid-5-amidacetat, oder in Form von Liposomen; und
eine therapeutisch wirksame Menge an Tripeptid-10-Citrullin, oder in Form von Liposomen;
zusammen mit einem oder mehreren pharmazeutisch akzeptablen Hilfsstoffen oder Trägersubstanzen;
oder ersatzweise,
(b) gleichzeitig, nacheinander oder getrennt:
(b1) einer pharmazeutischen Zusammensetzung umfassend eine therapeutisch wirksame Menge an Acetyl-sh-hexapeptid-5-amidacetat oder in Form von Liposomen; zusammen mit einem oder mehreren pharmazeutisch akzeptablen Hilfsstoffen oder Trägersubstanzen; und
(b2) einer pharmazeutischen Zusammensetzung umfassend eine therapeutisch wirksame Menge an Tripeptid-10-Citrullin, oder in Form von Liposomen; zusammen mit einem oder mehreren pharmazeutisch akzeptablen Hilfsstoffen oder Trägersubstanzen.

5. Das Acetyl-sh-hexapeptid-5-amidacetat, oder in Form von Liposomen, oder die pharmazeutische Zusammensetzung zur Verwendung nach einem der Ansprüche 1 bis 4 bei der Behandlung von Haarausfall, wobei die Behandlung durch Nachwachsen der Haare, durch Verhinderung von weiterem Haarausfall, durch Verringerung der Haarausfallrate, durch Verringerung der Telogenphase und Erhöhung der Anagenphase und/oder durch enzimatische Hemmung des Enzims 5-Alpha-Reduktase durchgeführt wird.

6. Kosmetische Verwendung von Acetyl-sh-hexapeptid-5-amidacetat, oder in Form von Liposomen; oder einer kosmetischen Zusammensetzung, die eine kosmetisch wirksame Menge an Acetyl-sh-hexapeptid-5-amidacetat, oder in Form von Liposomen zusammen mit einem oder mehreren kosmetisch akzeptablen Hilfsstoffen oder Trägern umfasst; als Haarpflegemittel bei einem Patienten, der nicht an einer Haarausfallstörung leidet, wobei die Haarpflege die Linderung von mindestens einem der folgenden Symptome umfasst: Haarsprödigkeit, Haarausfall, Haarbruch, mangelnder Haarelastizität, mangelnder Haarhelligkeit, mangelndem Haarvolumen, Haarrauhigkeit, Haarschuppenbildung, Haarentwässerung, Verringerung der Haardichte und Haartrockenheit.

7. Kosmetische Verwendung von Acetyl-sh-hexapeptid-5-amidacetat, oder in Form von Liposomen, als Haarpflegemittel bei einem Patienten, der nicht an einer Haarausfallstörung leidet; in Kombinationstherapie mit Tripeptid-10-Citrullin, oder in Form von Liposomen; wobei die Haarpflege die Linderung von mindestens einem der folgenden Symptome umfasst: Haarsprödigkeit, Haarausfall, Haarbruch, mangelnder Haarelastizität, mangelnder Haarhelligkeit, mangelndem Haarvolumen, Haarrauhigkeit, Haarschuppenbildung, Haarentwässerung, Verringerung der Haardichte und Haartrockenheit.

8. Kosmetische Verwendung von Acetyl-sh-hexapeptid-5-amidacetat, oder in Form von Liposomen; und Tripeptid-10-Citrullin, oder in Form von Liposomen, als Haarpflegemittel bei einem Patienten, der nicht an einer Haarausfallstörung leidet; wobei die Haarpflege die Linderung von mindestens einem der folgenden Symptome umfasst: Haarsprödigkeit, Haarausfall, Haarbruch, mangelnder Haarelastizität, mangelnder Haarhelligkeit, mangelndem Haarvolumen, Haarrauhigkeit, Haarschuppenbildung, Haarentwässerung, Verringerung der Haardichte und Haartrockenheit.

9. Die kosmetische Verwendung nach Anspruch 8, wobei die kosmetische Behandlung die Verabreichung an einen Patienten umfasst von:
entweder
(c) einer kosmetischen Zusammensetzung umfassend:
eine kosmetisch wirksame Menge an Acetyl-sh-Hexapeptid-5-amidacetat, oder in Form von Liposomen; und
eine kosmetisch wirksame Menge an Tripeptid-10-Citrullin oder in Form von Liposomen;
zusammen mit einem oder mehreren kosmetisch akzeptablen Hilfsstoffen oder Trägersubstanzen;
oder ersatzweise,
(d) gleichzeitig, nacheinander oder getrennt:
(d1) einer kosmetischen Zusammensetzung, umfassend eine kosmetisch wirksame Menge an Acetyl-sh-hexapeptid-5-amidacetat, oder in Form von Liposomen; zusammen mit einem oder mehreren kosmetisch akzeptablen Hilfsstoffen oder Trägersubstanzen; und
(d2) einer kosmetischen Zusammensetzung, umfassend eine kosmetisch wirksame Menge an Tripeptid-10-Citrullin oder in Form von Liposomen; zusammen mit einem oder mehreren kosmetisch akzeptablen Hilfsstoffen oder Trägersubstanzen.

10. Das Acetyl-sh-hexapeptid-5-amidacetat zur Verwendung nach einem der Ansprüche 1 bis 5: oder ersatzweise die kosmetische Verwendung nach einem der Ansprüche 6 bis 9, wobei die Behandlung einmal täglich verabreicht wird.

11. Eine pharmazeutische oder kosmetische Zusammensetzung umfassend:
eine therapeutisch oder kosmetisch wirksame Menge an Acetyl-sh-hexapeptid-5-Amidacetat, oder in Form von Liposomen; und
eine therapeutisch oder kosmetisch wirksame Menge an Tripeptid-10-Citrullin, oder in Form von Liposomen; zusammen mit einem oder mehreren pharmazeutisch oder kosmetisch akzeptablen Hilfsstoffen oder Trägersubstanzen.

12. Das Acetyl-sh-hexapeptid-5-amidacetat zur Verwendung nach einem der Ansprüche 1 bis 5: oder ersatzweise die kosmetische Verwendung nach einem der Ansprüche 6 bis 10; oder ersatzweise die pharmazeutische oder kosmetische Zusammensetzung nach Anspruch 11; wobei die pharmazeutische und kosmetische Zusammensetzung eine topische Zusammensetzung ist.

13. Das Acetyl-sh-hexapeptid-5-amidacetat zur Verwendung nach einem der Ansprüche 1 bis 5 und 12; oder ersatzweise die kosmetische Verwendung nach einem der Ansprüche 6 bis 10 und 12; oder ersatzweise die pharmazeutische oder kosmetische Zusammensetzung nach einem der Ansprüche 11 oder 12; wobei das Acetyl-sh-hexapeptid-5-amidacetat in Form von Liposomen und das Tripeptid-10-Citrullin in Form von Liposomen; vorzugsweise Lecithin-Liposomen, vorliegt.

14. Das Acetyl-sh-hexapeptid-5-amidacetat zur Verwendung nach einem der Ansprüche 1 bis 5 und 12 bis 13; oder ersatzweise die kosmetische Verwendung nach einem der Ansprüche 6 bis 10 und 12 bis 13; oder ersatzweise die pharmazeutische oder kosmetische Zusammensetzung nach einem der Ansprüche 11 bis 13; wobei:
der eine oder die mehreren pharmazeutisch oder kosmetisch akzeptablen Hilfsstoffe oder Trägerstoffe ausgewählt sind aus der Gruppe bestehend aus Vehikel, Dispergiermittel, Mitteln zur Kontrolle der Viskosität, pH-Einstellmittel, Konservierungsmittel, Antioxidans, Tensid, Emulgator und einer Mischung davon; und
die pharmazeutische oder kosmetische Zusammensetzung ferner einen oder mehrere zusätzliche pharmazeutische oder kosmetische Wirkstoffe umfasst; insbesondere ausgewählt aus der Gruppe bestehend aus einem Haarkonditionierungsmittel, einem Haut-/Kopfhautkonditionierungsmittel, einem Mittel gegen Haarausfall, Sonnenschutzmitteln, einem Mittel gegen Schuppen, einem für das Haar beruhigenden Mittel, einem Mittel gegen Seborrhoe, einem Haarverdichtungsmittel und einer Mischung davon.

15. Das Acetyl-sh-hexapeptid-5-amidacetat zur Verwendung nach einem der Ansprüche 1 bis 5 und 12 bis 14; oder ersatzweise die kosmetische Verwendung nach einem der Ansprüche 6 bis 10 und 12 bis 14; oder ersatzweise die pharmazeutische oder kosmetische Zusammensetzung nach einem der Ansprüche 11 bis 14; wobei: die wirksame Menge an Acetyl-sh-hexapeptid-5-amidacetat 0,001 bis 5 Gew.- % beträgt; und/oder die wirksame Menge an Tripeptid-10-Citrullin 0,001 bis 5 Gew.- % beträgt.

## Revendications

1. Acétate d'acétyl sh-hexapeptide-5 amide, ou sous forme de liposomes ;
ou une composition pharmaceutique comprenant la quantité thérapeutiquement efficace d'acétate d'acétyl sh-hexapeptide-5 amide, ou sous forme de liposomes avec un ou plusieurs excipients ou véhicules pharmaceutiquement acceptables ; pour son utilisation dans le traitement de la perte de cheveux chez un sujet souffrant d'un trouble de perte de cheveux.

2. Acétate d'acétyl sh-hexapeptide-5 amide, ou sous forme de liposomes, pour son utilisation dans le traitement de la perte de cheveux chez un sujet souffrant d'un trouble de la perte de cheveux, dans lequel l'acétate d'acétyl sh-hexapeptide-5 amide, ou sous forme de liposomes, est pour son utilisation en thérapie combinée avec le tripeptide-10 citrulline, ou sous forme de liposomes.

3. Acétate d'acétyl sh-hexapeptide-5 amide, ou sous forme de liposomes ; et tripeptide-10 citrulline, ou sous forme de liposomes, pour son utilisation dans le traitement de la perte de cheveux chez un sujet souffrant d'un trouble de perte de cheveux.

4. L'acétate d'acétyl sh-hexapeptide-5 amide, ou sous forme de liposomes ; et le tripeptide-10 citrulline, ou sous forme de liposomes, pour son utilisation selon la revendication 3, dans lequel le traitement comprend l'administration à un sujet de :
soit
(a) une composition pharmaceutique comprenant :
une quantité thérapeutiquement efficace d'acétate d'acétyl sh-hexapeptide-5 amide, ou sous forme de liposomes ; et
une quantité thérapeutiquement efficace de tripeptide-10 citrulline, ou sous forme de liposomes ;
avec un ou plusieurs excipients ou véhicules pharmaceutiquement acceptables ;
ou, en variante,
(b) simultanément, séquentiellement ou séparément :**
(b1) une composition pharmaceutique comprenant une quantité thérapeutiquement efficace d'acétate d'acétyl sh-hexapeptide-5 amide, ou sous forme de liposomes ; avec un ou plusieurs excipients ou véhicules pharmaceutiquement acceptables ; et
(b2) une composition pharmaceutique comprenant une quantité thérapeutiquement efficace de tripeptide-10 citrulline ou sous forme de liposomes ; avec un ou plusieurs excipients ou véhicules pharmaceutiquement acceptables.

5. L'acétate d'acétyl sh-hexapeptide-5 amide, ou sous forme de liposomes, ou la composition pharmaceutique pour son utilisation selon l'une quelconque des revendications 1 à 4 dans le traitement de la perte de cheveux dans lequel le traitement est effectué par la repousse des cheveux, par la prévention de la poursuite de la perte des cheveux, par la diminution du taux de perte de cheveux, par la réduction de la phase télogène et l'augmentation de la phase anagène et/ou par l'inhibition enzymatique de l'enzyme 5-alpha réductase.

6. Utilisation cosmétique de l'acétate d'acétyl sh-hexapeptide-5 amide, ou sous forme de liposomes ; ou d'une composition cosmétique comprenant une quantité cosmétiquement efficace d'acétate d'acétyl sh-hexapeptide-5 amide, ou sous forme de liposomes avec un ou plusieurs excipients ou véhicules cosmétiquement acceptables ; comme agent de soin des cheveux chez un sujet ne souffrant pas d'un trouble de la perte de cheveux, dans lequel le soin des cheveux comprend l'amélioration d'au moins l'un des symptômes suivants : fragilité des cheveux, amincissement des cheveux, rupture des cheveux, manque d'élasticité des cheveux, manque de brillance des cheveux, manque de volume des cheveux, rugosité des cheveux, écaillage des cheveux, déshydratation des cheveux, réduction de la densité des cheveux et sécheresse des cheveux.

7. Utilisation cosmétique de l'acétate d'acétyl sh-hexapeptide-5 amide, ou sous forme de liposomes, en tant qu'agent de soin des cheveux chez un sujet ne souffrant pas d'un trouble de la perte de cheveux ; en thérapie combinée avec le tripeptide-10 citrulline, ou sous forme de liposomes ; dans laquelle le soin des cheveux comprend l'amélioration d'au moins l'un des symptômes suivants : la fragilité des cheveux, l'amincissement des cheveux, la rupture des cheveux, la manque d'élasticité des cheveux, la manque de brillance des cheveux, la manque de volume des cheveux, la rugosité des cheveux, l'écaillage des cheveux, la déshydratation des cheveux, la réduction de la densité des cheveux et la sécheresse des cheveux.

8. Utilisation cosmétique d'acétate d'acétyle sh-hexapeptide-5 amide, ou sous forme de liposomes ; et de tripeptide-10 citrulline, ou sous forme de liposomes, en tant qu'agent de soin des cheveux chez un sujet ne souffrant pas d'un trouble de la perte de cheveux ; dans laquelle le soin des cheveux comprend l'amélioration d'au moins l'un des symptômes suivants : la fragilité des cheveux, l'amincissement des cheveux, la rupture des cheveux, la manque d'élasticité des cheveux, la manque de brillance des cheveux, la manque de volume des cheveux, la rugosité des cheveux, l'écaillage des cheveux, la déshydratation des cheveux, la réduction de la densité des cheveux et la sécheresse des cheveux.

9. L'utilisation cosmétique selon la revendication 8, dans laquelle le traitement cosmétique comprend l'administration à un sujet de :
soit
(c) une composition cosmétique comprenant :
une quantité cosmétiquement efficace d'acétate d'acétyl sh-hexapeptide-5 amide, ou sous forme de liposomes ; et
une quantité cosmétiquement efficace de tripeptide-10 citrulline ou sous forme de liposomes ;
avec un ou plusieurs excipients ou véhicules cosmétiquement acceptables ;
ou, en variante,
(d) simultanément, séquentiellement ou séparément :
(d1) une composition cosmétique comprenant une quantité cosmétiquement efficace d'acétate d'acétyl sh-hexapeptide-5 amide, ou sous forme de liposomes ; avec un ou plusieurs excipients ou véhicules cosmétiquement acceptables ; et
(d2) une composition cosmétique comprenant une quantité cosmétiquement efficace de tripeptide-10 citrulline ou sous forme de liposomes ; avec un ou plusieurs excipients ou véhicules cosmétiquement acceptables.

10. L'acétate d'acétyl sh-hexapeptide-5 amide pour son utilisation selon l'une quelconque des revendications 1 à 5 : ou, en variante, l'utilisation cosmétique selon l'une quelconque des revendications 6 à 9, dans laquelle le traitement est administré une fois par jour.

11. Une composition pharmaceutique ou cosmétique comprenant :
une quantité thérapeutiquement ou cosmétiquement efficace d'acétate d'acétyl sh-hexapeptide-5 amide, ou sous forme de liposomes ; et
une quantité thérapeutiquement ou cosmétiquement efficace de tripeptide-10 citrulline, ou sous forme de liposomes ;
avec un ou plusieurs excipients ou véhicules pharmaceutiquement ou cosmétiquement acceptables.

12. L'acétate d'acétyle sh-hexapeptide-5 amide pour son utilisation selon l'une quelconque des revendications 1 à 5 : ou, en variante, l'utilisation cosmétique selon l'une quelconque des revendications 6 à 10 ; ou, en variante, la composition pharmaceutique ou cosmétique selon la revendication 11 ; dans lequel la composition pharmaceutique et cosmétique est une composition topique.

13. L'acétate d'acétyl sh-hexapeptide-5 amide pour son utilisation selon l'une quelconque des revendications 1 à 5 et 12 ; ou, en variante, l'utilisation cosmétique selon l'une quelconque des revendications 6 à 10 et 12 ; ou, en variante, la composition pharmaceutique ou cosmétique selon l'une quelconque des revendications 11 ou 12 ; où l'acétate d'acétyl sh-hexapeptide-5 amide est sous forme de liposomes et le tripeptide-10 citrulline est sous forme de liposomes ; de préférence des liposomes de lécithine.

14. L'acétate d'acétyl sh-hexapeptide-5 amide pour son utilisation selon l'une quelconque des revendications 1 à 5 et 12 à 13 ; ou, en variante, l'utilisation cosmétique selon l'une quelconque des revendications 6 à 10 et 12 à 13 ; ou, en variante, la composition pharmaceutique ou cosmétique selon l'une quelconque des revendications 11 à 13 ; où :
le un ou les plusieurs excipients ou véhicules pharmaceutiquement ou cosmétiquement acceptables sont choisis dans le groupe constitué par un véhicule, un agent dispersant, un régulateur de la viscosité, un régulateur de pH, un conservateur, un antioxydant, un tensioactif, un émulsifiant et un mélange de ceux-ci ; et
la composition pharmaceutique ou cosmétique comprend en outre un ou plusieurs ingrédients actifs pharmaceutiques ou cosmétiques supplémentaires ; en particulier choisis dans le groupe constitué par un agent de conditionnement des cheveux, un agent de conditionnement de la peau/du cuir chevelu, un agent anti-perte des cheveux, les écrans solaires, un agent antipelliculaire, un agent apaisant pour les cheveux, un agent anti-séborrhée, un agent densifiant les cheveux et un mélange de ceux-ci.

15. L'acétate d'acétyl sh-hexapeptide-5 amide pour son utilisation selon l'une quelconque des revendications 1 à 5 et 12 à 14 ; ou, en variante, l'utilisation cosmétique selon l'une quelconque des revendications 6 à 10 et 12 à 14 ; ou, en variante, la composition pharmaceutique ou cosmétique selon l'une quelconque des revendications 11 à 14 ; où : la quantité efficace d'acétate d'acétyl sh-hexapeptide-5 amide est de 0,001 % à 5 % en poids ; et/ou la quantité efficace de tripeptide-10 citrulline est de 0,001 % à 5 % en poids.
